# EUROPEAN PATENT APPLICATION

(11) **EP 4 814 486 A1**
(43) Date of publication of application: **30.09.2026**
(21) Application number: 25166721.8
(22) Date of filing: 27.03.2025
(51) Int. Cl.: G01N 33/00, G01N 33/22

(54) **DEVICE FOR DETERMINING THE PURITIY OF A HYDROGEN GAS**

(71) Applicant: EMCEL GmbH, 50829 Köln (DE)
(72) Inventor: The designation of the inventor has not yet been filed
(74) Representative: Herzog IP Patentanwalts GmbH

(57) **Abstract**

A device for determining at least two chemical components of a gaseous composition, wherein the gaseous composition comprises at least 50 vol-% hydrogen, wherein the device comprises
a./ a first sensor that is adapted and arranged for measuring a content of a first chemical component in the gaseous composition, wherein the first chemical component is hydrogen;
b./ a computational unit;
wherein the computational unit is adapted and arranged to obtain a content of at least one even-further chemical component in the gaseous composition using at least one or all of the following: data stored in at least one database, an estimate, solving a mathematical equation, a combination of at least two thereof, and wherein said at least one even-further chemical component is not hydrogen.

## Description

### FIELD OF THE INVENTION

The invention pertains to a device for determining at least two chemical components of a gaseous composition, wherein the gaseous composition comprises at least 50 vol-% hydrogen. The invention further pertains to an apparatus that comprises a device according to the invention as an element of said apparatus. The invention further pertains to a method for determining at least two chemical components of a gaseous composition comprising at least 50 vol-% hydrogen. The invention further pertains to a use of a device according to the invention for determining a purity of a hydrogen gas. The invention also pertains to a computer program comprising instructions which, when the program is executed by a computational unit, cause the computational unit to carry out at least one step of the method according to the invention. The invention also pertains to a computer-readable storage medium comprising instructions having stored thereon a computer program according to the invention.

### BACKGROUND

The purity of hydrogen gas is of interest for a large number of applications. An example of such an application is the purity of hydrogen gas at a refuelling station for hydrogen-powered vehicles. Typically, the purity of hydrogen is tested by taking a sample at the refuelling station and sending the sample to a laboratory for analysis. However, the purity of the sample may not reflect the purity of the hydrogen that a user refuels their vehicle with. For example, following the taking of the sample, the hydrogen at the refuelling station is replenished by a provider. This replenished hydrogen may have a different purity compared to the purity of the sample.

### OBJECTS

An object of the present invention is to at least partially overcome at least one of the disadvantages encountered in the state of the art.

It is a further object of the invention to provide a device that allows for determining a larger number of chemical components in a gaseous composition.

It is a further object of the invention to provide a device, for determining the chemical components in a gaseous composition, that is easier to service.

It is a further object of the invention to provide a device, for determining the chemical components in a gaseous composition, that has a reduced build complexity.

It is a further object of the invention to provide a device, for determining the chemical components in a gaseous composition, that requires less energy.

It is a further object of the invention to provide a device, for determining the chemical components in a gaseous composition, that requires less time to determine said chemical components.

It is a further object of the invention to provide a device, for determining the chemical components in a gaseous composition, that has a reduced size.

It is a further object of the invention to provide a device, for determining the chemical components in a gaseous composition, that can be used at more locations and an in more vehicles.

It is a further object of the invention to provide a device, for determining the chemical components in a gaseous composition, that is more robust.

It is a further object of the invention to provide a device, for determining the chemical components in a gaseous composition, that is easier to use.

It is a further object of the invention to provide a device, for determining the chemical components in a gaseous composition, that has an improved integrability.

It is a further object of the invention to provide a device, for determining the chemical components in a gaseous composition, that allows for determining the chemical components on-site.

It is a further object of the invention to provide a device, for determining the chemical components in a gaseous composition, that allows for determining the chemical components continuously.

It is a further object of the invention to provide a device that allows for determining a purity of a hydrogen gas.

It is a further object of the invention to provide a method for determining a larger number of chemical components in a gaseous composition.

It is a further object of the invention to provide a method, for determining the chemical components in a gaseous composition, that requires less energy.

It is a further object of the invention to provide a method, for determining the chemical components in a gaseous composition, that requires less time to determine said chemical components.

It is a further object of the invention to provide a method, for determining the chemical components in a gaseous composition, that can be used at more locations and an in more vehicles.

It is a further object of the invention to provide a method, for determining the chemical components in a gaseous composition, that can be performed on-site.

It is a further object of the invention to provide a method, for determining the chemical components in a gaseous composition, that can be performed continuously.

It is a further object of the invention to provide a method for determining a purity of a hydrogen gas.

### PREFERRED EMBODIMENTS OF THE INVENTION

A contribution to at least partially fulfilling at least one of the above-mentioned objects is made by any of the embodiments of the invention.
A 1^{st} embodiment of the invention is a device for determining at least two chemical components of a gaseous composition, wherein the gaseous composition comprises at least 50 vol-%, preferably at least 70 vol-%, and more preferably at least 90 vol-% hydrogen, wherein the device comprises
a./ a first sensor that is adapted and arranged for measuring a content of a first chemical component in the gaseous composition, wherein the first chemical component is hydrogen;
b./ optionally a second sensor, preferably adapted and arranged to measure a content of a second chemical component;
c./ optionally a third sensor, preferably adapted and arranged to measure a content of a third chemical component;
d./ optionally one or more further sensors, preferably adapted and arranged to measure a content of at least one or more further chemical components;
e./ a computational unit;
wherein
the computational unit is adapted and arranged to obtain a content of at least one even-further chemical component in the gaseous composition using at least one or all of the following: data stored in at least one database, an estimate, solving a mathematical equation, a combination of at least two thereof, and
wherein
said at least one even-further chemical component is not hydrogen.

In an aspect of the 1^{st} embodiment, the vol-% of the hydrogen is based on a total volume of the gaseous composition. In a preferred aspect of the 1^{st} embodiment, the content of the at least one even-further chemical component is obtained using data stored in at least one database. In this preferred aspect of the 1^{st} embodiment, the device preferably comprises one or more storage mediums with the at least one database stored thereon. In a preferred aspect of the 1^{st} embodiment, the first chemical component is not measured using gas chromatograph. In a preferred aspect of the 1^{st} embodiment, the first chemical component is not measured using chromatography mass spectroscopy. In a preferred aspect of the 1^{st} embodiment, the first sensor is adapted and arrange to measure the content of the first chemical component by receiving the gaseous composition.

In a preferred embodiment of the device, the first sensor is selected from the group consisting of an electrical sensor, an electrochemical sensor, a thermal conductive sensor, an electromagnetic wave sensor, and a combination of at least two thereof. This preferred embodiment is a 2^{nd} embodiment of the invention, that preferably depends on the 1^{st} embodiment of the invention.

In one preferred aspect of the 2^{nd} embodiment, the first sensor is selected from the group consisting of an electrical sensor, an electrochemical sensor, a thermal conductive sensor, an electromagnetic wave sensor, and a combination of at least two thereof, wherein the electromagnetic sensor is not an infrared sensor. In another preferred aspect of the 2^{nd} embodiment, the first sensor is selected from the group consisting of an electrical sensor, an electrochemical sensor, a thermal conductive sensor, and a combination of at least two thereof. In yet another preferred aspect of the 2^{nd} embodiment, the first sensor is not an infrared sensor. In a preferred aspect of the 2^{nd} embodiment, the first sensor is a thermal conductive sensor.

In a preferred embodiment of the device, the device is adapted and arranged to measure a content of a second chemical component in the gaseous composition, wherein the second chemical component is not hydrogen. This preferred embodiment is a 3^{rd} embodiment of the invention, that preferably depends on any of the 1^{st} to 2^{nd} embodiments of the invention.

In one preferred aspect of the 3^{rd} embodiment, the first sensor is adapted and arranged to measure hydrogen and the second chemical component. In another preferred aspect of the 3^{rd} embodiment, the device further comprises the second sensor, wherein the first sensor is adapted and arranged to measure hydrogen and the second sensor is adapted and arranged to measure the second chemical component.

In a preferred embodiment of the device, the second chemical component is water (e.g., water in the gaseous phase) or oxygen (e.g., O₂). This preferred embodiment is a 4^{th} embodiment of the invention, that preferably depends on the 3^{rd} embodiment of the invention.

In a preferred embodiment of the device, the device comprises the second sensor, wherein the second sensor is adapted and arranged to measure the content of the second chemical component. This preferred embodiment is a 5^{th} embodiment of the invention, that preferably depends on any of the 3^{rd} to 4^{th} embodiments of the invention.

In one preferred aspect of the 5^{th} embodiment, the second sensor is adapted and arranged to measure the content of water, more preferably the content of water in the gaseous phase. In another preferred aspect of the 5^{th} embodiment, the second sensor is adapted and arranged to measure the content of oxygen, more preferably the content of O₂. In yet another preferred aspect of the 5^{th} embodiment, the second sensor is adapted and arranged to measure both the content of water and the content of oxygen, preferably both the content of water in the gaseous phase and the content of O₂. In a preferred aspect of the 5^{th} embodiment, the second sensor is adapted and arrange to measure the content of the second chemical component by receiving the gaseous composition.

In a preferred embodiment of the device, the second sensor is selected from the group consisting of an electrical sensor, an electrochemical sensor, a thermal conductive sensor, an electromagnetic wave sensor, a dew point sensor, and a combination of at least two thereof. This preferred embodiment is a 6^{th} embodiment of the invention, that preferably depends on the 5^{th} embodiment of the invention.

In a preferred embodiment of the device, the device is adapted and arranged to measure a content of a third chemical component in the gaseous composition, wherein the third chemical component is not hydrogen. This preferred embodiment is a 7^{th} embodiment of the invention, that preferably depends on any of the 1^{st} to 6^{th} embodiments of the invention.

In the 7^{th} embodiment, the third chemical component differs from the second chemical component. In one preferred aspect of the 7^{th} embodiment, the first sensor is adapted and arranged to measure the content of hydrogen and the content of the third chemical component. In another preferred aspect of the 7^{th} embodiment, the second sensor is adapted and arranged to measure the content of the second chemical component and the content of the third chemical component.

In a preferred embodiment of the device,
A.] the device is adapted and arranged to measure the content of the second chemical component in the gaseous composition, wherein the second chemical component is not hydrogen, and
B.] the second chemical component is water (e.g., water in the gaseous phase) and the third chemical component is oxygen (e.g., O₂).

This preferred embodiment is an 8^{th} embodiment of the invention, that preferably depends on the 7^{th} embodiment of the invention.

The 8^{th} embodiment should not be understood to mean that measuring the content of water is more preferred than measuring the content of oxygen. For example, in an alternative 8^{th} embodiment, the second chemical component is oxygen (e.g., O₂) and the third chemical component is water (e.g., water in the gaseous phase).

In a preferred embodiment of the device, the device comprises the third sensor, wherein the third sensor is adapted and arranged to measure the content of the third chemical component. This preferred embodiment is an 9^{th} embodiment of the invention, that preferably depends on any of the 7^{th} to 8^{th} embodiments of the invention.

In a preferred aspect of the 9^{th} embodiment, the first sensor is adapted and arranged to measure the content of hydrogen, the second sensor is adapted and arranged to measure the content of the second chemical component, and the third sensors is adapted and arranged to measure the content of the third chemical component. In a preferred aspect of the 9^{th} embodiment, the third sensor is adapted and arrange to measure the content of the third chemical component by receiving the gaseous composition.

In a preferred embodiment of the device, the third sensor is selected from the group consisting of an electrical sensor, an electrochemical sensor, a thermal conductive sensor, an electromagnetic wave sensor, a dew point sensor, and a combination of at least two thereof. This preferred embodiment is a 10^{th} embodiment of the invention, that preferably depends on the 9^{th} embodiment of the invention.

In a preferred embodiment of the device, the device comprises the second sensor and the third sensor, wherein
A.] the second sensor is adapted and arranged for measuring the content of water (e.g., water in the gaseous phase) in the gaseous composition, and
B.] the third sensor is adapted and arranged for measuring the content of oxygen (e.g., O₂) in the gaseous composition.

This preferred embodiment is an 11^{th} embodiment of the invention, that preferably depends on any of the 1^{st} to 10^{th} embodiments of the invention, and more preferably on any of the 7^{th} to 10^{th} embodiments of the invention.

The 11^{th} embodiment should not be understood to mean that measuring the content of water is more preferred than measuring the content of oxygen. For example, in an alternative 11^{th} embodiment, the second sensor is adapted and arranged for measuring the content of oxygen (e.g., O₂) and the third sensor is adapted and arranged for measuring the content of water (e.g., water in the gaseous phase).

In a preferred embodiment of the device, the device is adapted and arranged to measure a content of one or more further chemical components in the gaseous composition, and wherein the one or more further chemical components differ from hydrogen. This preferred embodiment is an 12^{th} embodiment of the invention, that preferably depends on any of the 1^{st} to 11^{th} embodiments of the invention.

In the 12^{th} embodiment, the one or more further chemical components differ from the second chemical component and the third chemical component.

In a preferred embodiment of the device, the one or more further chemical components are at least one or all of the following: nitrogen gas, ammonia, at least one sulphur-containing compound (e.g., hydrogen sulphide, sulphur dioxide, and ethanethiol), formic acid, formaldehyde, at least one halogen-containing compound (e.g., dichloromethane), at least one hydrocarbon-containing compound (e.g., methane, ethane, benzene, compounds comprising up to 12 carbon atoms, complex hydrocarbons, oils, and fats), carbon monoxide, carbon dioxide, at least one noble gas (e.g., helium and argon). This preferred embodiment is a 13^{th} embodiment of the invention, that preferably depends on the 12^{th} embodiment of the invention.

In a preferred aspect of the 13^{th} embodiment, the one or more further chemical components are at least one or all of the following: carbon monoxide, carbon dioxide, methane. In one preferred aspect of the 13^{th} embodiment, the first sensor is adapted and arranged to measure the content of hydrogen and the content of at least one or more further chemical components. In another preferred aspect of the 13^{th} embodiment, the second sensor is adapted and arranged to measure the content of the second chemical component and the content of the at least one or more further chemical components. In yet another preferred aspect of the 13^{th} embodiment, the third sensor is adapted and arranged to measure the content of the third chemical component and the content of the at least one or more further chemical components.

In a preferred embodiment of the device, the device comprises the one or more further sensors, wherein the one or more further sensors are adapted and arranged for measuring the content of the one or more further chemical components. This preferred embodiment is a 14^{th} embodiment of the invention, that preferably depends on any of the 12^{th} to 13^{th} embodiments of the invention.

In a preferred aspect of the 14^{th} embodiment, the first sensor is adapted and arranged to measure the content of hydrogen, the second sensor is adapted and arranged to measure the content of the second chemical component, the third sensors is adapted and arranged to measure the content of the third chemical component, and the one or more further sensors are adapted and arranged to measure the content of the one or more further chemical components. In a preferred aspect of the 14^{th} embodiment, the one or more further sensors are adapted and arrange to measure the content of the one or more further chemical components by receiving the gaseous composition.

In a preferred embodiment of the device, the one or more further sensors are selected from the group consisting of an electrical sensor, an electrochemical sensor, a thermal conductive sensor, an electromagnetic wave sensor, a dew point sensor, and a combination of at least two thereof. This preferred embodiment is a 15^{th} embodiment of the invention, that preferably depends on the 14^{th} embodiment of the invention.

In a preferred aspect of the 15^{th} embodiment, at least one sensor, of the one or more further sensors, is an infrared sensor.

In a preferred embodiment of the device, one of the following applies:
a. the device is adapted and arranged to obtain a content of nitrogen in the gaseous composition using at least one or all of the following: data stored in at least one database, an estimate, solving a mathematical equation, a combination of at least two thereof; and more preferably without using a sensor that is adapted and arranged for measuring a content of nitrogen; or
b. the device comprises a sensor that is adapted and arranged to measure the content of nitrogen in the gaseous composition.

This preferred embodiment is a 16^{th} embodiment of the invention, that preferably depends on any of the 1^{st} to 15^{th} embodiments of the invention.

In a preferred aspect of the 16^{th} embodiment, feature a., the device does not comprise a sensor that is adapted and arranged to measure the content of nitrogen.

In a preferred embodiment of the device, the device has a maximum dimension that is 1 m or less, preferably 0.8 m or less, more preferably 0.6 m or less, and further preferably 0.4 m or less. This preferred embodiment is a 17^{th} embodiment of the invention, that preferably depends on any of the 1^{st} to 16^{th} embodiments of the invention.

In the 17^{th} embodiment examples of a maximum dimension include a width, a length, and a height. The "maximum dimension" in the 17^{th} embodiment should preferably be understood as the dimension of the device with the largest measured value.

In a preferred embodiment of the device, the device is adapted and arranged to allow for the gaseous composition to flow through the device, thereby allowing for the determining of the at least two chemical components of the gaseous composition. This preferred embodiment is an 18^{th} embodiment of the invention, that preferably depends on any of the 1^{st} to 17^{th} embodiments of the invention.

In a preferred embodiment of the device, the device is adapted and arranged for at least one or all of the following:
a. to allow for a flow of the gaseous composition through the device at a rate of 0.2 to 2 Nl/min, preferably from 0.4 to 1.7 Nl/min, more preferably from 0.4 to 1.4 Nl/min, and further preferably from 0.8 to 1.2 Nl/min;
b. determining the at least two chemical components of the gaseous composition at an overpressure that is at least 0.1 bar, preferably at least 1 bar, more preferably at least 10 bar, and further preferably at least 100 bar.

This preferred embodiment is an 19^{th} embodiment of the invention, that preferably depends on any of the 1^{st} to 18^{th} embodiments of the invention.

In an aspect of the 19^{th} embodiment, all possible combinations of the features a. and b. are preferred aspects of the embodiment. These combinations are e.g., a; b; a+b. In the 19^{th} embodiment, "overpressure" should preferably be understood as the pressure in excess of atmospheric pressure. In a preferred aspect of the 19^{th} embodiment, feature a., the flow rate is in the range of 0.9 to 1.1 Nl/min. In a preferred aspect of the 19^{th} embodiment, feature b. should preferably be understood to mean that the "overpressure" is the overpressure of the gaseous composition in the device. In the 19^{th} embodiment, feature b., the overpressure may be as high as 1000 bar.

In a preferred embodiment of the device, the computational unit is in data communication with at least one or all of the following: the first sensor, the second sensor, if present, the third sensor, if present, the one or more further sensors, if present. This preferred embodiment is a 20^{th} embodiment of the invention, that preferably depends on any of the 1^{st} to 19^{th} embodiments of the invention.

In a preferred embodiment of the device, the device comprises at least one means of informing a user of at least one chemical component present in the gaseous composition (e.g., a display means, such as a screen, an audio alert, such as an alarm sound, a visual means, such as a light being activated). This preferred embodiment is a 21^{st} embodiment of the invention, that preferably depends on any of the 1^{st} to 20^{th} embodiments of the invention.

In a preferred embodiment of the device, the device comprises an algorithm that is adapted and arranged to at least partially determine a chemical composition of the gaseous composition. This preferred embodiment is a 22^{nd} embodiment of the invention, that preferably depends on any of the 1^{st} to 21^{st} embodiments of the invention.

In a preferred aspect of the 22^{nd} embodiment, the algorithm is adapted and arranged to determine at least one, more preferably at least two, chemical components of the gaseous composition. In a preferred aspect of the 22^{nd} embodiment, the algorithm is adapted and arranged to at least partially determine a purity of the hydrogen content in the gaseous composition. In a preferred aspect of the 22^{nd} embodiment, the algorithm is adapted and arranged to perform at least one of the steps of the method as described in any of the 28^{th} to 39^{th} embodiments of the invention. In this aspect, it is preferred that the algorithm is adapted and arranged to perform at least step C./ in the 28^{th} embodiment of the invention and/or one or more of the steps of the 29^{th} to 39^{th} embodiments of the invention.

In a preferred embodiment of the device, the algorithm is adapted and arranged to at least partially determine the chemical composition using at least one or all of the following:
a. data stored in at least one database;
b. input from at least one or all of the following: the first sensor, the second sensor, if present, the third sensor, if present, the one or more further sensors, if present.

This preferred embodiment is a 23^{rd} embodiment of the invention, that preferably depends on the 22^{nd} embodiment of the invention.

In an aspect of the 23^{rd} embodiment, all possible combinations of the features a. and b. are preferred aspects of the embodiment. These combinations are e.g., a; b; a+b. In a particularly preferred aspect of the 23^{rd} embodiment, the algorithm is adapted and arranged to at least partially determines the chemical composition using data stored in at least one database.

In a preferred embodiment of the device, the device, more preferably the computational unit, is adapted and arranged to obtain the content of the at least one even-further chemical component in the gaseous composition without using a sensor that is adapted and arranged for measuring the content of said at least one even-further chemical component. This preferred embodiment is a 24^{th} embodiment of the invention, that preferably depends on any of the 1^{st} to the 23^{rd} embodiments of the invention.

In a preferred embodiment of the device, the computational unit is adapted and arranged to obtain the content of the at least one even-further chemical component in the gaseous composition using an estimate based on at least one probability, preferably at least one probability stored in at least one database. This preferred embodiment is a 25^{th} embodiment of the invention, that preferably depends on any of the 1^{st} to the 24^{th} embodiments of the invention.

In a preferred aspect of the 25^{th} embodiment, the at least one probability is one of the following: a probability that an even-further chemical component is present in a gaseous composition as such, or the probability that the even-further chemical component is present with a specific content.

In a preferred embodiment of the device, each chemical component of the gaseous composition is composed of a plurality of the same atoms or a plurality of the same molecules, wherein at least one atom or molecule of each chemical component of the gaseous composition is provided simultaneously to the first sensor. This preferred embodiment is a 26^{th} embodiment of the invention, that preferably depends on any of the 1^{st} to the 25^{th} embodiments of the invention.

In a preferred aspect of the 26^{th} embodiment, at least one, more preferably at least two chemical components are not at least partially removed from the gaseous composition prior to measuring the content of the first chemical component.

In a preferred embodiment of the device, the device is adapted and arranged to determine the at least two chemical components of the gaseous composition in 90 second or less, preferably 45 seconds or less, more preferably 35 seconds or less, even more preferably 25 seconds or less, even more preferably 15 seconds or less, further preferably 10 seconds or less, and even further preferably 5 seconds or less. This preferred embodiment is a 27^{th} embodiment of the invention, that preferably depends on any of the 1^{st} to the 26^{th} embodiments of the invention.

A 28^{th} embodiment of the invention is a method, more preferably a computer-implemented method, for determining at least two chemical components of a gaseous composition comprising at least 50 vol-%, preferably at least 70 vol-%, and more preferably at least 90 vol-% hydrogen, the method comprising the steps of
A./ optionally, providing a device according to the invention, preferably a device according to any of 1^{st} to 27^{th} embodiments of the invention, and preferably feeding the gaseous composition through the device;
B./ measuring, in the gaseous composition,
   i. a content *X_{H2}* of a first chemical component, preferably using the first sensor, wherein the first chemical component is hydrogen,
   ii. optionally a content *X₂* of a second chemical component, preferably using the second sensor,
   iii. optionally a content *X₃* of a third chemical component, preferably using a third sensor,
   iv. optionally, a content *X_{Fi}* of one or more further chemical components, preferably using one or more further sensors, wherein *X_{Fi}* is the content of an ith further chemical component, with *i =* 1 ... *n,* and *n* is the number of further chemical components;
C./ calculating a first difference *Y₁ =* 1 - ∑ *G_{J}Z_{J},* where the sum is over *J* = 1 ... *m,* with *m* ≥ 1 and *m* being the number of chemical components measured, with
   i.] *Z_{J}* representing the content of the chemical components measured, with *Z₁ =X_{H2},* and
   ii.] *G_{J}* > 0 being numerical factors, wherein preferably *G_{J}* = 1 for all *J= 1* ... *m;*
   wherein
   the measurement(s) in step B./ is/are performed using at least one sensor, preferably using the device according to any of the 1^{st} to 27^{th} embodiments of the invention.

In the 28^{th} embodiment, the second chemical component, the third chemical component and the one or more further chemical components are not hydrogen, and the second chemical component, the third chemical component, and the one or more further chemical components are different from each other. In the 28^{th} embodiment, if only the content of the first chemical component is measured, then the first difference in step C./ is calculated as *Y₁ =* 1 *- G₁X_{H2}.* In the 28^{th} embodiment, if the content of the first chemical component and the second chemical component are measured, then the first difference in step C./ is calculated as *Y₁ =* 1 *- G₁X_{H2} - G₂X₂.* In the 28^{th} embodiment, if the content of the first chemical component, the second chemical component, and the third chemical component are measured, then the first difference in step C./ is calculated as *Y₁ =* 1 *- G₁X_{H2} - G₂X₂ - G₃X₃.* In the 28^{th} embodiment, if the content of the first chemical component, the second chemical component, and one or more further chemical components are measured, then the first difference in step C./ is calculated as *Y₁ =* 1 *- G₁X_{H2} - G₂X₂ -* ∑*G₍₁₊₂₎X_{Fi},* wherein the sum is over *i =* 1 ... *n.* In the 28^{th} embodiment, if the content of the first chemical component, the second chemical component, the third chemical component, and one or more further chemical components are measured, then the first difference in step C./ is calculated as *Y₁* = 1 *- G₁X_{H2} - G₂X₂ - G₃X₃ -* Σ *G₍ᵢ₊₃₎X_{Fi},* wherein the sum is over *i =* 1 ... *n.* In a preferred aspect of the 28^{th} embodiment, the method is performed using a device that comprises at least one sensor and at least one computational unit, wherein the device is more preferably according to any of the 1^{st} to 27^{th} embodiments of the invention.

In a preferred embodiment of the method for determining at least two chemical components of a gaseous composition, the content *X₂* of the second chemical component is measured, wherein the second chemical component is water (e.g., water in the gaseous phase) or oxygen (e.g., O₂). This preferred embodiment is a 29^{th} embodiment of the invention, that preferably depends on the 28^{th} embodiment of the invention.

In a preferred embodiment of the method for determining at least two chemical components of a gaseous composition,
a./ the content of the first chemical component, the second chemical component, and the third chemical component are measured in step B./ of the 28^{th} embodiment of the invention, and
b./ the first difference in step C./ of the 28^{th} embodiment of the invention is calculated as follows: *Y₁ =* 1 *- G₁X_{H2} - G₂X₂- G₃X₃,* wherein preferably *G₁ = G₂ = G₃ = 1.*

This preferred embodiment is a 30^{th} embodiment of the invention, that preferably depends on any of the 28^{th} to 29^{th} embodiments of the invention.

In a preferred embodiment of the method for determining at least two chemical components of a gaseous composition, the second chemical component is water and the third chemical component is oxygen. This preferred embodiment is a 31^{st} embodiment of the invention, that preferably depends on the 30^{th} embodiment of the invention.

The 31^{st} embodiment should not be understood to mean that measuring the content of water is more preferred than measuring the content of oxygen. For example, in an alternative 31^{st} embodiment, the second chemical component is oxygen (e.g., O₂) and the third chemical component is water (e.g., water in the gaseous phase).

In a preferred embodiment of the method for determining at least two chemical components of a gaseous composition,
a./ the content of the first chemical component, the second chemical component, the third chemical component, and at least one or more further chemical components are measured in step B./ of the 28^{th} embodiment of the invention, and
b./ the first difference in step C./ of the 28^{th} embodiment of the invention is calculated as follows: *Y₁ =* 1 *- G₁X_{H2} - G₂X₂- G₃X₃ -* Σ *G₍ᵢ₊₃₎X_{Fi},* wherein the sum is over *i =* 1 ... *n,* wherein preferably *G₁ = G₂ = G₃ = G₍ᵢ₊₃₎ =* 1 for *all i =* 1 ... *n.*

This preferred embodiment is a 32^{nd} embodiment of the invention, that preferably depends on any of the 28^{th} to 29^{th} embodiments of the invention.

In one preferred aspect of the 32^{nd} embodiment, the second chemical component is water (e.g., water in the gaseous phase) and the third chemical component is oxygen (e.g., O₂). In another preferred aspect of the 32^{nd} embodiment, the second chemical component is oxygen (e.g., O₂) and the third chemical component is water (e.g., water in the gaseous phase). In a preferred aspect of the 32^{nd} embodiment, the one or more further chemical components include at least one or all of the following: carbon monoxide, carbon dioxide, and methane.

In a preferred embodiment of the method for determining at least two chemical components of a gaseous composition, if *Y₁ > T₁,* where *T₁* > 0 is a first threshold value, then the method further comprises the steps of adjusting the value of at least one of the numerical factors *G_{J}* and recalculating the first difference *Y₁* using the at least one adjusted *G_{J}*. This preferred embodiment is a 33^{rd} embodiment of the invention, that preferably depends on any of the 28^{th} to 32^{nd} embodiments of the invention.

In the 33^{rd} embodiment, a numerical factor *G_{J}* may be adjusted to take into account, e.g., a measurement accuracy of a sensor, atmospheric conditions (e.g., temperature, pressure), and a location of the measurement. In the 33^{rd} embodiment a numerical factor *G_{J}* may be adjusted by either increasing or decreasing the value of the numerical factor.

In a preferred embodiment of the method for determining at least two chemical components of a gaseous composition, the method further comprises the step of obtaining a content of at least one even-further chemical component of the gaseous composition using at least one or all of the following: data stored in at least one database, an estimate, solving a mathematical equation, a combination of at least two thereof. This preferred embodiment is a 34^{th} embodiment of the invention, that preferably depends on any of the 28^{th} to 33^{rd} embodiments of the invention.

In the 34^{th} embodiment, an even-further chemical component is different from hydrogen, the second chemical component, the third chemical component, and the at least one or more further chemical components. In a preferred aspect of the 34^{th} embodiment, the content of the at least one even-further chemical component is obtained by reading data stored in at least one database. In a preferred aspect of the 34^{th} embodiment, the content of the at least one even-further chemical component is obtained if *Y₁ > T₁,* where *T₁* > 0 is the first threshold value.

In a preferred embodiment of the method for determining at least two chemical components of a gaseous composition, the content of the at least one even-further chemical component in the gaseous composition is obtained without measuring the content of said at least one even-further chemical component using a sensor. This preferred embodiment is a 35^{th} embodiment of the invention, that preferably depends on the 34^{th} embodiment of the invention.

In a preferred embodiment of the method for determining at least two chemical components of a gaseous composition, *T₁ =* 10⁻¹, preferably *T₁* = 5 x 10⁻², more preferably *T₁ =* 10⁻², and further preferably *T₁* = 5 x 10⁻³. This preferred embodiment is a 36^{th} embodiment of the invention, that preferably depends on any of the 33^{rd} to 35^{th} embodiments of the invention.

In a preferred embodiment of the method for determining at least two chemical components of a gaseous composition, the content of the at least one even-further chemical component is estimated using at least one probability, preferably at least one probability stored in at least one database. This preferred embodiment is a 37^{th} embodiment of the invention, that preferably depends on any of the 28^{th} to 36^{th} embodiments of the invention.

In a preferred aspect of the 37^{th} embodiment, the at least one probability is one of the following: a probability that an even-further chemical component is present in a gaseous composition as such, or the probability that the even-further chemical component is present with a specific content.

In a preferred embodiment of the method for determining at least two chemical components of a gaseous composition, each chemical component of the gaseous composition is composed of a plurality of the same atoms or a plurality of the same molecules, wherein at least one atom or molecule of each chemical component of the gaseous composition is provided simultaneously to the first sensor. This preferred embodiment is a 38^{th} embodiment of the invention, that preferably depends on any of the 28^{th} to 37^{th} embodiments of the invention.

In a preferred aspect of the 38^{th} embodiment, at least one, more preferably at least two chemical components are not at least partially removed from the gaseous composition prior to measuring the content of the first chemical component.

In a preferred embodiment of the method for determining at least two chemical components of a gaseous composition, the at least two chemical components are determined in 90 second or less, preferably 45 seconds or less, more preferably 35 seconds or less, even more preferably 25 seconds or less, even more preferably 15 seconds or less, further preferably 10 seconds or less, and even further preferably 5 seconds or less. This preferred embodiment is a 39^{th} embodiment of the invention, that preferably depends on any of the 28^{th} to 38^{th} embodiments of the invention.

The 39^{th} embodiment should preferably be understood to mean that the steps of the method for determining at least two chemical components are performed within a limit time interval.

A 40^{th} embodiment of the invention is a device, preferably a device according to any of the 1^{st} to 27^{th} embodiments of the invention, comprising means for carrying out a method for determining at least two chemical components of a gaseous composition comprising at least 50 vol-%, preferably at least 70 vol-%, and more preferably at least 90 vol-% hydrogen, wherein said method is preferably the method according to any of the 28^{th} to 39^{th} embodiments of the invention.

In the 40^{th} embodiment, the "method" should be understood as a method according to the invention. In the 40^{th} embodiment, examples of the "means for carrying out the method" include the following: one or more sensors for measuring a content of a chemical component in the gaseous composition, a computational unit, a combination thereof.

A 41^{st} embodiment of the invention is a computer program comprising instructions which, when the program is executed by a computational unit, cause the computational unit to carry out at least one step of a method, according to the invention, for determining at least two chemical components of a gaseous composition comprising at least 50 vol-%, preferably at least 70 vol-%, and more preferably at least 90 vol-% hydrogen, wherein said method is preferably the method according to any of the 28^{th} to 39^{th} embodiments of the invention.

In a preferred aspect of the 41^{st} embodiment, the at least one step carried out by the computational unit is at least step C./ of the 28^{th} embodiment of the invention. In a preferred aspect of the 41^{st} embodiment, the instructions are adapted and arranged to control the measuring as performed in step B./ of the 28^{th} embodiment, e.g., by controlling the sensors used for the measurements. In a preferred aspect of the 41^{st} embodiment, the instructions are adapted and arranged to perform any of the steps as described in the 29^{th} to 39^{th} embodiments of the invention.

A 42^{nd} embodiment of the invention is a computer-readable storage medium having stored thereon a computer program according to the invention, preferably the computer program according to the 41^{st} embodiment of the invention.

A 43^{rd} embodiment of the invention is an apparatus comprising a device, according to the invention, as an element of said apparatus, wherein said device is preferably a device according to any of the 1^{st} to 27^{th} embodiments of the invention.

In a preferred aspect of the 43^{rd} embodiment, the apparatus comprises the computer program according to the 41^{st} embodiment of the invention. In a preferred aspect of the 43^{rd} embodiment, the apparatus comprises the computer-readable storage medium according to the 42^{nd} embodiment of the invention.

In a preferred embodiment of the apparatus, the apparatus is selected from the group consisting of a filling station, a refuelling station (e.g., a refuelling station for hydrogen-powered vehicles), a vehicle (e.g., a land-based vehicle, an aerial vehicle, a water-based vehicle), a pipeline, a hydrogen production facility, a hydrogen storage vessel, a plant (e.g., chemical plant), a container (e.g., a compressor container). This preferred embodiment is a 44^{th} embodiment of the invention, that preferably depends on the 43^{rd} embodiment of the invention.

A 45^{th} embodiment of the invention is a use of a device, according to the invention, for determining a purity of a hydrogen gas, wherein said device is preferably a device according to any of the 1^{st} to 27^{th} embodiments of the invention.

In a preferred aspect of the 45^{th} embodiment, the hydrogen gas comprises at least 50 vol-%, more preferably at least 70 vol-%, and further preferably at least 90 vol-% hydrogen, where the vol-% is based on a total volume of the hydrogen gas.

In a preferred embodiment of the use, the purity of the hydrogen gas is measured in at least one or all of the following: a filling station, a refuelling station (e.g., a refuelling station for hydrogen-powered vehicles), a vehicle (e.g., a land-based vehicle, an aerial vehicle, a water-based vehicle), a pipeline, a hydrogen production facility, a hydrogen storage vessel, a plant (e.g., chemical plant), a container (e.g., a compressor container). This preferred embodiment is a 46^{th} embodiment of the invention, that preferably depends on the 45^{th} embodiment of the invention.

In a preferred aspect of the invention, the device according to any of the 1^{st} to 27^{th} embodiments of the invention is used to perform the method according to any of the 28^{th} to 39^{th} embodiments of the invention.

### DETAILED DESCRIPTION OF THE INVENTION

Throughout this document, disclosures of ranges should preferably be understood to include both end points of the range. Furthermore, each disclosure of a range in the document should preferably be understood as also disclosing preferred sub-ranges in which one end point is excluded or both end points are excluded. For example, a disclosure of a range from *X₁* to *X₂* is to be understood as disclosing a range that includes both of the end points *X₁* and *X₂.* Furthermore, it is to be understood as also disclosing a range that includes the end point *X₁* but excludes the end point *X₂*, a range that excludes the end point*X₁* but includes the end point *X₂,* and a range that excludes both end points *X₁* and *X₂.*

Some preferred embodiments and preferred aspects have various combinations of features as alternatives. Where these various combinations are disclosed, the combinations are separated by a semi-colon (";"). For example, the list of the combination of features "a; a+b; a+c+d" for a preferred embodiment discloses a preferred embodiment that comprises the feature "a", a preferred embodiment that comprises the features "a" and "b", and a preferred embodiment that comprises the features "a", "c", and "d".

### Gaseous composition

Different chemical components should preferably be understood to have different chemical formulae. A chemical component should preferably be understood as being composed of a plurality the same atoms or a plurality of the same molecules.

Apart from hydrogen, examples of chemical components that may be present in the gaseous composition include one or more of the following: nitrogen gas (N₂); ammonia (NH₃); sulphur--containing compounds such as hydrogen sulphide (H₂S), sulphur dioxide (SO₂) and ethanethiol (CH₃CH₂SH; also known as ethyl mercaptan); formic acid (HCOOH); formaldehyde (CH₂O); halogen-containing compounds such as dichloromethane (CH₂Cl₂; also known as DCM, methylene chloride, or methylene bichloride); water (H₂0), e.g., water in the gaseous phase; hydrocarbon-containing compounds such as methane (CH₄), ethane (C₂H₆), benzene (C₆H₆), compounds comprising up to 12 carbon atoms, compounds comprising more than 12 carbon atoms, complex hydrocarbons, oils, and fats; oxygen gas (O₂); carbon monoxide (CO); carbon dioxide (CO₂); and noble gases such as helium gas (He) and argon gas (Ar).

Apart from hydrogen, the device is preferably adapted and arranged to determine the content of one or more of the chemical components as disclosed in, e.g., the norms ISO 14687:2025-02 and DIN EN 17124:2022-12.

A preferred gaseous composition comprises at least 50 vol-%, more preferably at least 60 vol-%, even more preferably at least 70 vol-%, and further preferably at least 80 vol-% hydrogen, with the vol-% based on a total volume of the gaseous composition. A more preferred gaseous composition comprises at least 90 vol-%, more preferably at least 92 vol-%, even more preferably at least 94 vol-%, and further preferably at least 96 vol-% hydrogen, with the vol-% based on a total volume of the gaseous composition. A preferred gaseous composition may comprise up to 100 vol-% hydrogen, with the vol-% based on a total volume of the gaseous composition.

The chemical components present in a gaseous composition are generally all in the gas phase. However, this is not a requirement. One or more chemical components present in the gaseous composition may also be in the solid phase (e.g., in the form of particulates) and/or in the liquid phase. An example of water in the gaseous phase is water vapour.

### Determining

The wording "determining" should preferably be understood as including at least one or all of the following: (a) measuring a content of at least one chemical component using a sensor that is adapted and arranged for this purpose, (b) obtaining a content of at least one chemical component using at least one or all of the following: data stored in at least one database, an estimate, solving a mathematical equation, a combination of at least two thereof, (c) a combination of (a) and (b).

The wording "determining at least two chemical components of a gaseous composition" should preferably be understood to mean that at least hydrogen is determined, and possibly at least one further chemical component of the gaseous composition. For example, if the gaseous composition consists of hydrogen (i.e., hydrogen makes up 100 vol-% of the gaseous composition), then only hydrogen is determined. For example, if the gaseous composition comprises less than 100 vol-% hydrogen, then hydrogen and at least one other chemical component is determined.

The wording "determining at least two chemical components of a gaseous composition" should preferably not be understood to mean that the content of all chemical components possibly present in the gaseous composition are necessarily determined. While this may be the case, there may also be chemical components present in the gaseous composition that are not determined.

A content of a chemical component that is measured should preferably be understood as being measured using a sensor. A content of a chemical component that is measured using a sensor is preferably measured by the sensor receiving the gaseous composition. Here "receiving the gaseous composition" should be understood as including at least one or all of the following: by the gaseous composition flowing through the sensor, by the gaseous composition flowing past the sensor, by the gaseous composition flowing over the sensor, a combination of at least two thereof.

The wording "the gaseous composition is provided simultaneously to a sensor" should preferably be understood to mean that the gaseous composition is provided to said sensor, thereby allowing said sensor to receive the gaseous composition.

If a content of a chemical component is measured, this should not be understood to mean that said chemical component is necessarily present in the gaseous composition. For example, a content of water in the gaseous composition is measured, where the content is measured to be 0 vol-%.

**In** at least one preferred embodiment, "the content of at least one even-further chemical component in the gaseous composition is obtained without using a sensor that is adapted and arranged for measuring the content of said at least one even-further chemical component". This should preferably not be understood to mean that no sensor measurements are used for obtaining the content of the at least one even-further chemical component. For example, measurements made using, e.g., a temperature sensor, a pressure gauge and/or a hygrometer may be used to for obtaining the content of the at least one even-further chemical component.

A content of a chemical component may be measured by at least partially removing said chemical component from the gaseous composition prior to the measurement. Here the partial removal may be done using e.g., a separation column and/or a filter.

### Hydrogen

The hydrogen present in the gaseous composition is preferably present in the form of a gas, i.e., H₂. A sensor that is adapted and arranged for measuring the content of hydrogen is preferably adapted and arranged for measuring the content of hydrogen gas.

**In** a preferred aspect of the invention, the first chemical component is not measured using gas chromatograph. **In** a preferred aspect of the invention, the first chemical component is not measured using chromatography mass spectroscopy.

### Content

A "content" of a chemical component should preferably be understood as, e.g., a number, a mass, a volume, a concentration, or a combination of at least two thereof, of the chemical component in the gaseous composition.

The content of a chemical component can be measured in any units that the skilled person deems suitable for the present invention. Examples of these units include wt-%, ppm, ppm wt, ppm volume, and vol-%. A value expressed in wt-% should preferably be understood to be based on the total weight of the gaseous composition. A value expressed in vol-% should preferably be understood to be based on the total volume of the gaseous composition.

### Sensors

A "sensor adapted and arranged for measuring a content" should preferably be understood to include any means and/or unit that is adapted and arranged to measure a content of at least one chemical component in a gaseous composition. Sensors adapted and arranged for measuring a content, and which are suitable for the present invention are preferably selected from the Group S, which has the following group members: a mass spectrometer, a gas chromatograph, a sensor adapted and arranged for chromatography mass spectroscopy (GC-MS), a sensor adapted and arrange for infrared spectroscopy, a sensor adapted and arranged for Fourier transform infrared spectroscopy, an electrical sensor, an electrochemical sensor, a thermal conductive sensor, an electromagnetic wave sensor, a dew point sensor, an acoustic sensor (e.g., sound sensor), a flame ionisation detector, an electron capture detector, a photo-ionisation detector, and combinations of at least two thereof. Example of an electrical sensor include a capacitive sensor and a resistive sensor. Examples of an electromagnetic wave sensor include an infrared sensor and an optical sensor (i.e., a sensor adapted and arranged to detect light in the visible spectrum).

**In** a preferred embodiment of the invention, the first sensor is selected from the Group S. **In** a preferred embodiment of the invention, the second sensor is selected from the Group S. **In** a preferred embodiment of the invention, the third sensor is selected from the Group S. **In** a preferred embodiment of the invention, at least one sensor, of the at least one or more further sensors, is selected from the Group S.

A sensor that is adapted and arranged for measuring a content of a specific chemical component should not necessarily be understood to mean that said sensor is adapted and arranged for only measuring the content of said specific chemical component. For example, where it is disclosed that a sensor is adapted and arranged to measure a content of a second chemical component, said sensor may also be adapted and arranged to measure a content of one or more further chemical components. For example, it may be disclosed that a sensor is adapted and arranged to measure a content of CO. Said sensor may also be adapted and arranged to measure the content of CO₂ and CH₄ in addition to the content of CO. However, a sensor may also be adapted and arranged to measure a content of only a single chemical component of the gaseous composition.

A first sensor is adapted and arranged for measuring a content of hydrogen. This should not necessarily be understood to mean that the first sensor is adapted and arranged for measuring only the content of hydrogen. For example, the first sensor may also be adapted and arranged to measure a content of at least one other chemical component other than hydrogen. In a preferred embodiment of the invention, the first sensor is not a gas chromatograph, or a sensor adapted and arranged for chromatography mass spectroscopy (GC-MS).

In a preferred embodiment of the invention, at least one sensor of the device (e.g., the first sensor, the second sensor, the third sensor) is not an infrared sensor. A sensor that is not an infrared sensor should preferably be understood to mean that the sensor does not measure electromagnetic waves in the infrared range (between 780 nm and 1 mm).

In a preferred embodiment of the invention, each chemical component of the gaseous composition is composed of a plurality of the same atoms or a plurality of the same molecules, wherein at least one atom or molecule of each chemical component of the gaseous composition is provided simultaneously to at least one or both of the following: the second sensor, the third sensor.

Any sensor that the skilled person deems suitable for the present invention may be used. Suitable sensors are commercially available from a number of vendors. Examples of a hydrogen sensor are the H2 Hydrogen Sensor, commercially available from Stange Elektronik GmbH (Germany), and the MGC^{Hydrogen} sensor, commercially available from Meter-Q Solutions GmbH (Germany). An example of a water sensor is the DewP sensor commercially available from Pro-Chem-Analytik GmbH Co. KG (Germany). An example of an oxygen sensor is the EC900 sensor commercially available from APM Technik GmbH (Germany). An example of an infrared sensor is the INFRA.sens AK250 sensor commercially available from Wi.Tec - Sensorik GmbH (Germany). The aforementioned infrared sensor is capable of measuring, amongst others, CO, CO₂, and CH₄.

### Computational unit

A computational unit comprises at least one processing means. A preferred computational unit comprises at least one storage medium. A preferred computational unit is adapted and arranged for communication, e.g., using LAN, Wi-Fi, and/or Bluetooth.

An example of a processing means is an integrated circuit. Examples of an integrated circuit include a processing unit, a memory unit, an application-specific integrated circuit, a microcontroller, a field-programmable gate array, and a system-on-chip. Examples of a processing unit include a central processing unit (CPU), a graphics processing unit (GPU), an XPU (X-processing unit), a tensor processing unit (TPU), a neuromorphic processing unit (NPU).

A storage medium as referred to herein should preferably be understood as a computer-readable storage medium. Example of a storage medium include random access memory (RAM), flash memory and a hard drive.

Two components of the device that are in "data communication" should preferably be understood to mean that data can be communicated between said components. Here an example of the two components is a sensor and a computational unit.

### Probability of even-further chemical component

A probability that an even-further chemical component is present in a gaseous composition may be a based on, e.g., an ambient pressure, an ambient temperature, a measurement accuracy of one or more of the sensors of the device (e.g., the first sensor, the second sensor), a supply chain of the gaseous composition, a supplier of the gaseous composition, the method used to produce the gaseous composition, the method used to transport the gaseous composition, a method used to purify the gaseous composition, a method used to store the gaseous composition, the coordinates where the device according to the invention is used.

A probability that an even-further chemical component is present in a gaseous composition may be the probability that the even-further chemical component is present as such and/or the probability that the even-further chemical component is present with a specific content.

For example, the device is used to determine at least two chemical components of a gaseous composition, wherein said gaseous composition was produced using a first production method and wherein the device is operated in an environment with a first ambient temperature. For this first production method and the first ambient temperature, the database contains a first probability that the gaseous composition comprises hydrogen sulphide with a specific content. For this first production method and a further ambient temperature, the database also contains a further probability that the gaseous composition comprises hydrogen sulphide with the specific content, wherein the first probability differs from the further probability.

### Database

A database according to the invention may comprises statistical data. Examples of statistical data is the probability that an even-further chemical component is present in a gaseous composition. Additionally and/or alternatively, a database according to the invention may comprise data that can be used to calculate the probability that an even-further chemical component is present in a gaseous composition. Additionally and/or alternatively, a database according to the invention may comprise data that is neither statistical data nor data that can be used for determining a probability. For example, the database may comprise data related to the impurity content of a gaseous composition, as provided by the producer of said gaseous composition.

### Further aspects of the device

In one preferred embodiment, the device comprises at least one means of informing a user of at least one of the chemical components present in the gaseous composition. Examples of said at least one means of informing the user include a display means (such as a screen), an audio alert (such as an alarm sound) a visual means (such as a light being activated), and a signal to a central control system.

A device according to the invention may also comprise one or more further measurement means. Examples of further measurement means include a thermometer, a pressure gauge, a hygrometer, a barometer, a flow meter.

In one preferred embodiment, the device is adapted and arranged for determining the at least two chemical components of the gaseous composition continuously. Here "continuously" should preferably be understood to mean that the device can determine the at least two chemical components at pre-defined time intervals (e.g., every hour or every 10 seconds), more preferably without requiring user input (e.g., automatically).

### Further aspects of the method

In one or more preferred embodiments, the first threshold value T₁ is predefined. Here "predefined" should preferably be understood to mean that a threshold value is set prior to performing a measurement using one or more sensors.

In a preferred aspect of the invention, the method for determining at least two chemical components of a gaseous composition, comprising at least 50 vol-% hydrogen, further comprises the step of displaying the results of the determination to a user, e.g., by means of a screen. For example, said method comprises the step of displaying to the user the content of the chemical components that were determined in the gaseous composition.

The invention is now illustrated by non-limiting examples and exemplifying embodiments.

### FIGURES

### List of figures

The figures serve to exemplify the present invention and should not be viewed as limiting the invention. Furthermore, the figures are not drawn to scale.
- Fig. 1:: schematic illustration of a device, according to the invention, for determining at least two chemical components of a gaseous composition that comprises at least 50 vol-% hydrogen.
- Fig. 2:: flow diagram of a method, according to the invention, for determining at least two chemical components of a gaseous composition that comprises at least 50 vol-% hydrogen.

### Description of figures

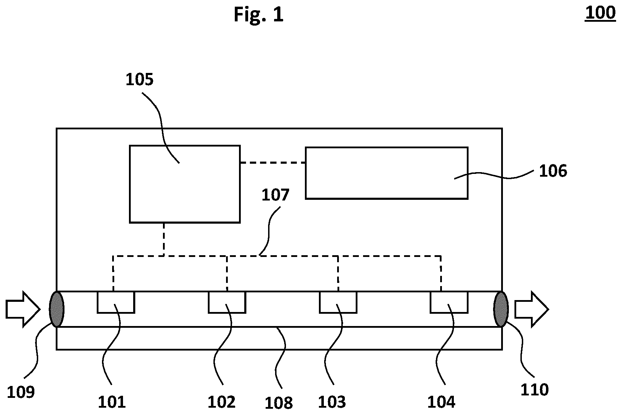

Fig. 1 shows a schematic illustration of a device 100, according to the invention, for determining at least two chemical components of a gaseous composition that comprises at least 50 vol-% hydrogen. The gaseous composition may be, e.g., a hydrogen gas that comprises at least 97 vol-% H₂. The device 100 can thus be used to measure the purity of the hydrogen gas.

As shown in Fig. 1, the device 100 has a circulation pipeline 108 that allows for the gaseous composition to flow through the device 100. The circulation pipeline 108 has a first opening 109 where the gaseous composition can enter the circulation pipeline 108. The circulation pipeline 108 also has a further opening 110 where the gaseous composition can exit the circulation pipeline 108.

The device 100 further comprises a first sensor 101 that is adapted and arranged to measure a content of a first chemical component in the gaseous composition flowing through the circulation pipeline 108. The device 100 also comprises a second sensor 102 that is adapted and arranged to measure a content of a second chemical component, a third sensor 103 that is adapted and arranged to measure a content of a third chemical component, and a further sensor 104 that is adapted and arranged to measure a content of a number of further chemical components in the gaseous composition flowing through the circulation pipeline 108. In Fig. 1, the first chemical component is hydrogen gas (H₂), the second chemical component is water in the gaseous phase, the third chemical component is oxygen gas (O₂), and the further chemical components are carbon monoxide (CO) and carbon dioxide (CO₂). In Fig. 1, the first sensor 101 is a thermal conductive sensor, the second sensor 102 is a dew point sensor, the third sensor 103 is an electrochemical sensor, and the further sensor 104 is an infrared sensor. For measuring the content of the first, second and third chemical components, these chemical components are not separated out of the gaseous composition prior to measuring (separation may be done with, e.g., a separation column, as used by gas chromatographs).

The device 100 in Fig. 1 further comprises a computational unit 105. This computational unit 105 comprises a processing means (such as a CPU) and a storage medium. The computational unit 105 is adapted and arranged to obtain a content of at least one even-further chemical component in the gaseous composition using data stored in a database (in turn stored on the storage medium). For example, measurements taken by the sensors 101, 102, 103, and 104 indicate that the content (e.g., vol-%) measured for the hydrogen, water, oxygen, carbon monoxide, and carbon dioxide do not add up to a selected threshold value (e.g., 99.99 vol-%). The computational unit 105 then reads data from a database to determine the probability that at least one or more even-further chemical components are present in the gaseous composition with a specific content. For example, the data indicates the probability that methane (CH₄) is present in the gaseous composition with a content of 100 to 120 ppm. The device therefore determines the chemical components present in the gaseous composition using both measurements and probabilities.

The computational unit 105 is also in data communication with the sensors 101, 102, 103, and 104 by means of the data connection 107. The computational unit 105 also comprises an algorithm (e.g., stored on the storage medium) that can be executed by the computational unit 105 (e.g., using the processing means). This algorithm is used to at least partially determine the chemical components present the gaseous composition using input from the first sensor 101, the second sensor 102, the third sensor 103, and the further sensor 104 (i.e., using the measurements taken by these sensors) as well as data from the database. The device 100 may also comprises, e.g., a display means 106 (e.g., an LCD screen) that can provide the user with information regarding, e.g., a content of a respective chemical component (such as the first chemical component) present in the gaseous composition and/or a purity of the gaseous composition.

It should be kept in mind that Fig. 1 is a simplified illustration of a device according to the invention. A device according to the invention may have any number of additional components, such as valves, pressure regulators, circuitry, a thermometer, and a pressure gauge. The device may also comprise sensors, in addition to sensors 101 to 104, for measuring the content of other chemical components that are not measured by the sensors 101 to 104, such as nitrogen. An example of an additional sensor that may also be present in the device 100 is a gas chromatograph. The circulation pipeline 108 may also be different to that shown in Fig. 1.

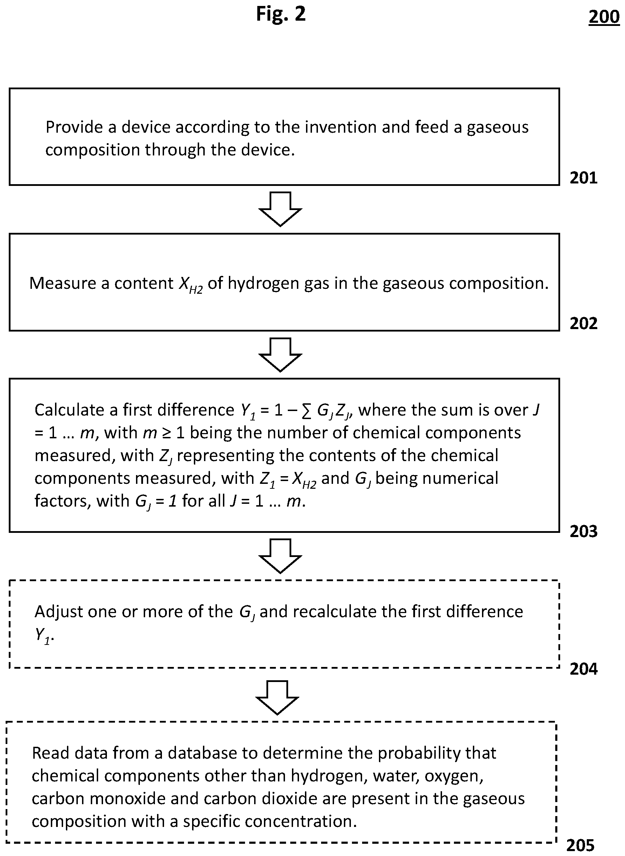

Fig. 2 is a flow diagram showing the steps of a method 200, according to the invention, for determining at least two chemical components of a gaseous composition that comprises at least 50 vol-% hydrogen. The method 200 is a computer implemented method.
- Step 201:: the device 100 according to Fig. 1 is provided and a gaseous composition is fed through the device.
- Step 202:: the following chemical components are measured in the gaseous composition: the first sensor 101 is used to measure the content *X_{H2}* of hydrogen gas (the first chemical component), the second sensor 102 is used to measure the content *X₂* of water (the second chemical component), the third sensor 103 is used to measure the content *X₃* of oxygen gas (the third chemical component), and the further sensor 104 is used to measure the content *X_{F1}* of carbon monoxide and the content *X_{F2}* of carbon dioxide (the further chemical components).
- Step 203:: the measurements of the sensors taken in step 203 are provide to the computational unit 105, which uses the algorithm to calculate a first difference *Y₁ =* 1 *- G₁X_{H2} - G₂X₂ - G₃X₃ - G₄X_{F1} - G₅X_{F2},* where initially *G₁ = G₂ = G₃ = G₄ = G₅ =* 1. If *Y₁* ≤ *T₁,* the content of hydrogen gas, water, oxygen gas, carbon monoxide and carbon dioxide present in the gaseous composition are displayed on the display means 106. Here *T₁* is a first threshold value that may be set based on, e.g., a desired accuracy of the measurement performed by the device 100 and/or the purity requirements for the gaseous composition. For example, *T₁ =* 10⁻². However, if *Y₁ > T₁,* step 204 is performed.
- Step 204:: one or more of the *G_{J}* values, e.g., *G₃,* may optionally be adjusted (increased or decreased), followed by recalculating the first difference *Y₁ = 1 - G₁X_{H2} - G₂X₂ - G₃X₃ - G₄X_{F1} - G₅X_{F2}* using the adjusted *G_{J}* value(s). This adjustment may be to take into account, e.g., atmospheric conditions at the location where the measurement is made. If it is again calculated that *Y₁ > T₁,* then step 205 is performed. Here *Y₁ > T₁* indicates that one or more chemical components (e.g., methane), other than those measured in step 202, may be present in the gaseous composition.
- Step 205:: data is read from one or more databases. This data is used to determine the probability that chemical components other than hydrogen, water, oxygen, carbon monoxide and carbon dioxide are present in the gaseous composition with a specific content.

In an alternative embodiment of the method shown in Fig. 2, if it is found in step 203 that *Y₁ > T₁,* then the method proceeds directly to step 205 (i.e., step 204 is not performed).

### EXAMPLES

The invention is illustrated further by way of examples. The invention is not restricted to the examples.

### Example 1.1

A first device, as described in Fig. 1, is provided. A gaseous composition comprising at least 50 vol-% hydrogen is split into a first fraction and a second fraction, with the first fraction fed through the first device. Using the first device, the content of a number of chemical components in said first fraction of the gaseous composition is determined. In particular, the first device is used to measure the content (e.g., vol-%) of hydrogen in the first fraction of the gaseous composition (i.e., the first device comprises a hydrogen sensor). The first device determines the chemical components in the first fraction of the gaseous composition based on measurements made by the sensors of the first device, as well as using data stored in a database.

### Example 1.2

A second device that comprises two gas chromatographs is provided. The second fraction of the gaseous composition (cf. Example 1.1) is fed through the second device. Using the second device, the content of a number of chemical components in said second fraction of the gaseous composition is determined. In contrast to the first device, the second device is not adapted and arranged to measure the content (e.g., vol-%) of hydrogen in the second fraction of the gaseous composition (i.e., the second device does not have a hydrogen sensor). The second device determines the chemical components in the second fraction of the gaseous composition solely based on the measurements made by the two gas chromatographs.

Table 1 is a comparison of the first device (Example 1.1) and the second device (Example 1.2) with respect to technical effects of said devices.

**Table 1**

| | Ex. 1.1 | Ex. 1.2 |
|---|---|---|
| **Technical effect** | | |
| Number of chemical components determinable | + | - |
| Serviceability | + | - |
| Build complexity | - | + |
| Energy requirements | - | + |
| Speed of measurement | - | + |
| Size of device | - | + |
| Adaptability | + | - |
| Robustness | + | - |
| Ease of use | + | - |
| Integrability | + | - |
| On-site | Yes | Yes |
| Continuous measurements | Yes | Yes |

The technical effects in Table 1 are as follows:
- Number of chemical components determinable: the number of chemical components in the gaseous composition that can be determined. A "+" indicates that more chemical components can be determined, while a "-" indicates that less chemical components can be determined. It is desired to increase the number of chemical components that can be determined.
- Serviceability: how easy it is to service and repair the device. A "+" indicates that it is easier to repair the device, while a "-" indicates that is more difficult to repair the device. It is desired that it is easier to repair the device.
- Build complexity: for example, the number of electronic components and circuits required for the device. A "-" indicates that less electronic components and circuits are required, while a "+" indicates that more electronic components and circuits are required. It is desired to reduce the number of electronic components and circuits that are required.
- Energy requirements: the amount of energy required by the device to determine the chemical components of the gaseous composition. A "-" indicates that less energy is required, while a "+" indicates that more energy is required. It is desired to reduce the energy that is required.
- Speed of measurement: the time required to determine the chemical components of the gaseous composition. A "-" indicates that less time is required, while a "+" indicates that more time is required. It is desired to reduce the time required to determine said chemical components.
- Size of device: a "-" indicates a smaller device, while a "+" indicates a larger device. It is desired to reduce the size of the device.
- Adaptability: the number of locations and vehicles where the device can be used. A "+" indicates that the device can be used at more locations and in more vehicles, while a "-" indicates that the device can be used at less locations and in less vehicles. It is desired to increase the number of locations and vehicles where the device can be used.
- Robustness: the device's resistance to damage. A "+" indicates that the device is more robust, while a "-" indicates that the device is less robust. It is desired to increase the robustness.
- Ease of use: how easy it is to use the device. A "+" indicates that the device is easier to use, while a "-" indicates that the device is less easy to use. It is desired to increase the ease of use.
- Integrability: how easy it is to integrate the device into a circulation system for the gaseous composition. For example, whether the device can be integrated into a hydrogen pump at a hydrogen refuelling station for hydrogen-powered vehicles. A "+" indicates that it is easier to integrate the device, while a "-" indicates that it is less easy to integrate the device. It is desired to increase the integrability.
- On-site: whether the determination of the chemical components of the gaseous composition can be performed on-site (e.g., the transport of a test sample of the gaseous composition to a laboratory for further analysis is not an on-site measurement). On-site measurements are desired.
- Continuous measurements; whether the device can automatically and continually measure the chemical components of the gaseous composition at set time-intervals. It is desired that the device can make continuous measurements.

### Example 2

Tables 2 and 3 below provide examples of the data tables that may be used to estimate the probability that a specific even-further chemical component is present in the gaseous composition.

Table 2 shows the probability that CH₄ is present in the gaseous composition with a content of 100 ppm, based on the method used to produce the gaseous composition. For example, if the gaseous composition is produced using Method A, then there is a larger probability that CH₄ is present with a content of 100 ppm, compared to Methods B and C.

Method A is the production of H₂ as a by-product of a chemical industrial process, Method B is the production of H₂ using electrolysis, and Method C is the production of H₂ from reforming natural gas.

**Table 2**

| Probability of CH₄ present with content of 100 ppm | |
|---|---|
| **Production method** | **Probability [%]** |
| Method A | 90 |
| Method B | 45 |
| Method C | 54 |

Table 3 shows the probability that CH₄ is present in the gaseous composition with a content of 100 ppm, based on the method of transporting the gaseous composition. For example, if the gaseous composition is transported via pipeline, then there is a larger probability that CH₄ is present with a content of 100 ppm, compared to when the gaseous composition is transported using a tank truck.

**Table 3**

| Probability of CH₄ present with content of 100 ppm | |
|---|---|
| **Transport method** | **Probability [%]** |
| Pipeline | 30 |
| Vehicle (tank truck) | 15 |

The above tables are merely examples. For example, a table with probabilities may be a multi-dimension table (e.g., Tables 2 and 3 may be combined).

When determining the chemical components present in the gaseous composition, e.g., the ten even-further chemical components with the highest probability may be selected. If the sum of the content of the measured chemical components and the content of these ten even-further chemical components do not add up to 100 vol-%, then a new set of ten, even-further chemical components may be selected. This procedure may be repeated until a vol-% of 100 % is calculated. Additionally or alternatively, e.g., a user may be provided with a list of all even-further chemical components, as well as the probability of a specific even-further chemical component being present.

The above examples are provided to illustrate the invention and should not be seen as limiting the invention. For example, the above probabilities may be used in different calculations to determine the content of at least one even-further chemical component.

### REFERENCE LIST

- 100: Device
- 101: First sensor
- 102: Second sensor
- 103: Third sensor
- 104: Further sensor
- 105: Computational unit
- 106: Display means
- 107: Data connection
- 108: Circulation pipeline
- 109: First opening
- 110: Further opening

## Claims

1. A device for determining at least two chemical components of a gaseous composition, wherein the gaseous composition comprises at least 50 vol-% hydrogen, wherein the device comprises
a./ a first sensor that is adapted and arranged for measuring a content of a first chemical component in the gaseous composition, wherein the first chemical component is hydrogen;
b./ optionally a second sensor, preferably adapted and arranged to measure a content of a second chemical component;
c./ optionally a third sensor, preferably adapted and arranged to measure a content of a third chemical component;
d./ optionally one or more further sensors, preferably adapted and arranged to measure a content of at least one or more further chemical components;
e./ a computational unit;
wherein
the computational unit is adapted and arranged to obtain a content of at least one even-further chemical component in the gaseous composition using at least one or all of the following: data stored in at least one database, an estimate, solving a mathematical equation, a combination of at least two thereof, and
wherein
said at least one even-further chemical component is not hydrogen.

2. The device according to claim 1, wherein, each chemical component of the gaseous composition is composed of a plurality of the same atoms or a plurality of the same molecules, wherein at least one atom or molecule of each chemical component of the gaseous composition is provided simultaneously to the first sensor.

3. The device according to any of the preceding claims, wherein the computational unit is adapted and arranged to obtain the content of the at least one even-further chemical component in the gaseous composition using an estimate based on at least one probability.

4. The device according to any of the preceding claims, where the device is adapted and arranged to obtain the content of the at least one even-further chemical component in the gaseous composition without using a sensor that is adapted and arranged for measuring the content of said at least one even-further chemical component.

5. The device according to any of the preceding claims, wherein the device has a maximum dimension that is 1 m or less.

6. The device according to any of the preceding claims, wherein the device comprises an algorithm that is adapted and arranged to at least partially determine a chemical composition of the gaseous composition.

7. A method for determining at least two chemical components of a gaseous composition comprising at least 50 vol-% hydrogen, the method comprising the steps of
A./ optionally, providing a device according to any of the preceding claims;
B./ measuring, in the gaseous composition,
i. a content *X_{H2}* of a first chemical component, wherein the first chemical component is hydrogen,
ii. optionally a content *X₂* of a second chemical component,
iii. optionally a content *X₃* of a third chemical component,
iv. optionally, a content *X_{Fi}* of one or more further chemical components, wherein *X_{Fi}* is the content of an ith further chemical component, *with i = 1* ... *n,* and *n* is the number of further chemical components;
C./ calculating a first difference *Y₁ =* 1 - ∑ *G_{J}Z_{J},* where the sum is over *J* = 1 ... *m,* with *m* ≥ 1 and *m* being the number of chemical components measured, with
i.] *Z_{J}* representing the content of the chemical components measured, with *Z₁ =X_{H2},* and
ii.] *G_{J}* > 0 being numerical factors, wherein preferably *G_{J}* = 1 for all *J= 1* ... *m;*
wherein
the measurement(s) in step B./ is/are performed using at least one sensor.

8. The method according to claim 7, wherein if *Y₁ > T₁,* where *T₁* > 0 is a first threshold value, then the method further comprises the steps of adjusting the value of at least one of the numerical factors *G_{J}* and recalculating the first difference *Y₁* using the at least one adjusted *G_{J}*.

9. The method according to any of the preceding claims 7 to 8, wherein, the method further comprises the step of obtaining a content of at least one even-further chemical component of the gaseous composition using at least one or all of the following: data stored in at least one database, an estimate, solving a mathematical equation, a combination of at least two thereof.

10. The method according to any of the preceding claims 7 to 9, wherein the content of the at least one even-further chemical component is estimated using at least one probability.

11. A device comprising means for carrying out the method according to any of the preceding claims 7 to 10.

12. A computer program comprising instructions which, when the program is executed by a computational unit, cause the computational unit to carry out at least one step of the method according to any of the preceding claims 7 to 10.

13. A computer-readable storage medium comprising instructions having stored thereon the computer program according to claim 12.

14. An apparatus comprising the device according to any of the claims 1 to 6 as an element of said apparatus.

15. A use of the device according to any of the claims 1 to 6 for determining a purity of a hydrogen gas.
